# EUROPÄISCHE PATENTSCHRIFT

(11) **EP 2 561 893 B1**
(45) Veröffentlichungstag und Bekanntmachung des Hinweises auf die Patenterteilung: **18.05.2016**
(21) Anmeldenummer: 12004697.4
(22) Anmeldetag: 22.06.2012
(51) Int. Cl.: A61L 2/10, E03C 1/126, C02F 1/32, E03F 5/04

(54) **Fußbodenabfluss mit UV-Strahler und Spülvorrichtung**
Floor drain with UV lamp and rinsing device
Écoulement au sol avez lampe UV et moyen de rinçage

(30) Priorität: 24.08.2011 DE 202011104771 U
(43) Veröffentlichungstag der Anmeldung: 27.02.2013
(73) Patentinhaber: BG Edelstahl und Kunststofftechnik für Krankenhaus Industrie und Wasserwirtschaft GmbH, 45739 Oer-Erkenschwick (DE)
(72) Erfinder: Barselak, Peter, 45739 Oer-Erkenschwick (DE)
(74) Vertreter: Gesthuysen Patent- und Rechtsanwälte

(56) Entgegenhaltungen:
- WO-A1-2011/079286
- JP-A- 2010 275 841
- US-A1- 2008 213 128
- DATABASE WPI Week 201142 Thomson Scientific, London, GB; AN 2011-D64705 XP002684837, & KR 2011 0032329 A (EUN R J) 30. März 2011 (2011-03-30) & KR 2011 0032329 A (JUN EUN RYEONG [KR]) 30. März 2011 (2011-03-30)

## Beschreibung

Die Erfindung betrifft einen Fußbodenabfluss zur Installation in einem Operationsraum und zur Abführung von den in einem Operationsraum anfallenden Flüssigkeiten, insbesondere von mit Krankheitserregern kontaminierten Flüssigkeiten.

Operationsräume müssen hohen Anforderungen an die Hygiene genügen, um die Wahrscheinlichkeit einer Infektion im Zusammenhang mit einer Operation sowohl des behandelten Patienten als auch des involvierten Operations- und Reinigungspersonals zu minimieren. Die Hygiene eines Operationsraums wird im Verlauf einer Operation durch das Anfallen von festen und flüssigen Stoffen wie Körpergewebe und -flüssigkeiten, die oftmals mit Krankheitserregern kontaminiert sind, beeinträchtigt. Daher ist es nach der Operation erforderlich, die Hygiene des Operationsraums für die nachfolgende Operation durch Reinigen und Desinfizieren unter Anwendung bewährter Verfahren und Mittel wiederherzustellen und unter anderem durch Personenschleusen und keimarme Luft bis zum Beginn der nachfolgenden Operation aufrechtzuerhalten.

Zur Umsetzung der Anforderungen an die Hygiene und zur Unterstützung der zuverlässigen und effizienten Reinigung und Desinfektion ist ein Operationsraum in besonderer Weise gestaltet. So ist beispielsweise der Bodenbelag eines Operationsraums völlig eben, abwaschbar, fugendicht, flüssigkeitsdicht und desinfektionsmittelresistent. Auch die in einem Operationsraum befindlichen Geräte sind zuverlässig zu reinigen und zu desinfizieren, weshalb deren Oberflächen vergleichbare Merkmale wie der Bodenbelag aufweisen. Schwer zu reinigende Geräte werden vor einer Operation mit keimfreien Hüllen vor Kontamination mit Krankheitserregern geschützt und die Hüllen nach der Operation entsorgt. Da bekannte Fußbodenabflüsse im Gegensatz zu dem Bodenbelag nur schwer zu reinigen und zu desinfizieren sind und daher in der Praxis oft vernachlässigt werden, wodurch es zu einer Beeinträchtigung der Hygiene und zu Geruchsbelastungen kommt, wird für gewöhnlich auf Fußbodenabflüsse in Operationsräumen verzichtet.

KR 2011/003 23 29 und JP 2010/275 841 offenbaren einen Fußbodenabfluss für medizinische Einrichtungen mit einer UV-Lampe zur Desinfektion und Desodorierung des Abflusses.

US 2008/213 128 offenbart einen Geruchsverschluss zur Anwendung in medizinischen Einrichtungen, welcher eine UV-Lampe zur Entkeimung umfasst.

Stand der Technik bei der Entsorgung von den in einem Operationsraum anfallenden Flüssigkeiten, wie zum Beispiel der während einer Operation bei einer Blasenspülung benutzten Spüllösung, ist vorzugsweise das weitestgehende Auffangen der Flüssigkeiten in Behältnissen und das Binden der verbleibenden, sich oftmals über den Bodenbelag verteilenden, Flüssigkeiten an Feststoffe. Mit Flüssigkeiten sind hier auch immer Flüssigkeiten eingeschlossen, die Feststoffe mit sich führen. Im Anschluss an die Operation erfolgt das Entleeren der Behältnisse in einem außerhalb des Operationsraums gelegenen Abfluss. Selbstverständlich sind auch diese Behältnisse und der benutzte Abfluss nach der Operation zu reinigen und zu desinfizieren. Die Behältnisse können alternativ entsorgt werden. Die derzeitige Art und Weise der Entsorgung von Flüssigkeiten ist nicht nur anfällig für Mängel an der Hygiene, sondern sie stellt auch ein zusätzliches Infektionsrisiko für das Reinigungspersonal dar, das die Entsorgung vornimmt, und ist aufwändig in der Durchführung.

Aufgabe der vorliegenden Erfindung ist die Schaffung einer Möglichkeit, die in einem Operationsraum anfallenden Flüssigkeiten, insbesondere die mit Krankheitserregern kontaminierten Flüssigkeiten, unmittelbar, insbesondere ohne Zwischenlagerung in Behältnissen, und ohne Beeinträchtigung der Hygiene oder eine durch die Erfindung bedingte erforderliche wesentliche Erhöhung des Reinigungs- und / oder Desinfektionsaufwands aus dem Operationsraum abzuführen.

Die zuvor hergeleitete und aufgezeigte Aufgabe ist durch den Gegenstand des Anspruchs 1 gelöst. Der erfindungsgemäß ausgestaltete Fußbodenabfluss ist in der Lage, die in Operationsräumen anfallenden Flüssigkeiten abzuführen und genügt dabei gleichzeitig durch die Reinigungswirkung der Spülvorrichtung und die Desinfektionswirkung der Bestrahlungsvorrichtung zusammenwirkend mit dem Gasverschluss den Anforderungen an die Hygiene in Operationsräumen. Darüber hinaus unterbindet der Gasverschluss eine Geruchsbelastung durch die mit dem Ablauf des Fußbodenabflusses verbunden Leitungen.

Die Desinfektion des durch die Einleitung von den Flüssigkeiten mit Krankheitserregern kontaminierten Innenraums des Behälters erfolgt durch Ultraviolettlicht. Ultraviolettlicht inaktiviert Krankheitserreger durch Zerstörung von Nukleinsäuren und wirkt auf diese Weise keimtötend, wobei die Wirksamkeit von der Intensität des Ultraviolettlichts abhängt. Die desinfizierende Wirkung des erfindungsgemäßen Fußbodenablaufs erstreckt sich folglich auf Krankheitserreger, die durch Ultraviolettlicht inaktiviert werden. Die Spülvorrichtung reinigt durch Abspülen den Innenraum und insbesondere den Ultraviolettlicht emittierenden Teil der Bestrahlungseinrichtung von Rückständen, die zuvor in den Flüssigkeiten gelöst waren oder mitgeführt wurden. Ohne die Reinigung durch Abspülen des Ultraviolettlicht emittierenden Teils der Bestrahlungsvorrichtung nähme die Desinfektionswirkung durch die sukzessive Anlagerung von Rückständen und dem damit einhergehenden Abfall der Strahlungsintensität ab.

Der erfindungsgemäße Fußbodenabfluss bietet den Vorteil einer Abführung der in einem Operationsraum anfallenden Flüssigkeiten, insbesondere von mit Krankheitserregern kontaminierten Flüssigkeiten, unmittelbar in diesem Operationsraum. Dabei bleibt der Aufwand für die Reinigung und Desinfektion des mit dem erfindungsgemäßen Fußbodenabfluss ausgestatteten Operationsraums zur Wiederherstellung der Hygiene nach einer Operation im Vergleich zu einem Operationsraum ohne den erfindungsgemäßen Fußbodenabfluss im Wesentlichen gleich. Das Auffangen und Zwischenlagern in Behältnissen entfällt und damit auch das Reinigen und Desinfizieren oder alternativ das Entsorgen der Behältnisse. Auch entfällt der Transport zu einem geeigneten Abfluss außerhalb des Operationsraums und das Entleeren der Behältnisse in diesen Abfluss mit den damit einhergehenden Risiken für die Hygiene und das ausführende Personal.

Bei einer ganz besonders bevorzugten Ausgestaltung der Erfindung ist der Gasverschluss als ein Geruchsverschluss ausgebildet. Die durch den Geruchsverschluss im Innenraum des Behälters verbleibenden Flüssigkeiten trennen den Innenraum des Behälters vom Ablauf hermetisch, wodurch eine Geruchsbelastung aus den mit dem Ablauf verbundenen Leitungen unterbunden wird. Der Innenraum des Behälters wird von in den Flüssigkeiten enthaltenen Rückständen durch die Spülvorrichtung gereinigt und die im Behälter verbleibenden Flüssigkeiten werden durch die Bestrahlungsvorrichtung desinfiziert.

Bei einer weiteren bevorzugten Ausgestaltung sind sowohl die Spülvorrichtung als auch die Bestrahlungsvorrichtung im Innenraum des zylinderförmigen Behälters angeordnet, wobei die Spülvorrichtung eine Spülmittelzuführung, eine Spülmittelverteilung und zumindest einen Spülmittelaustritt umfasst und wobei die Bestrahlungsvorrichtung eine elektrische Ultraviolettlichtquelle und eine Energiezuführung umfasst. Die äußere Form des Fußbodenablaufs entspricht durch die Anordnung sowohl der Spülvorrichtung als auch der Bestrahlungsvorrichtung im Innenraum des Behälters der Form des Behälters, wobei durch die ununterbrochene geometrische Form des Fußbodenablaufs die Installation des Fußbodenablaufs erleichtert wird.

Eine bevorzugte Realisierung des erfindungsgemäßen Fußbodenablaufs umfasst einen lösbar mit dem Behälter verbindbaren Deckel, wobei die Verbindung zwischen Deckel und Behälter durch ein Dichtmittel hermetisch dicht, insbesondere dicht gegenüber in Operationsräumen anfallenden Flüssigkeiten und Desinfektionsmitteln, ist; der Deckel ist damit Bestandteil des Behälters. Nach Entfernung des Deckels sind die im Innenraum des Behälters angeordneten Komponenten wie Spülvorrichtung, Bestrahlungsvorrichtung und Gasverschluss zugänglich.

In einer weiteren bevorzugten Ausgestaltung ist im Innenraum des Behälters ein Sensor angeordnet, der die von der Ultraviolettlichtquelle emittierte und auf den Sensor einfallende Intensität des Ultraviolettlichts misst. Da die Intensität des von der Ultraviolettlichtquelle emittierten Ultraviolettlichts aus verschiedenen Gründen, zum Beispiel durch Alterung der Ultraviolettlichtquelle, abnehmen kann, sinkt auch die Desinfektionswirkung, weshalb es erforderlich ist, die Ultraviolettlichtquelle bei Unterschreitung einer bestimmten Intensität auszutauschen. Eine Abnahme der Intensität durch Rückstände auf der Ultraviolettlichtquelle wird durch die Spülvorrichtung verhindert. Die Ultraviolettlichtquelle ist nach Entfernung des Deckels zugänglich und kann ausgetauscht werden.

In einer weiteren bevorzugten Ausgestaltung der Erfindung ist eine Leitung für die Zuführung der Flüssigkeiten in den Fußbodenabfluss mit dem Zulauf durch eine Verbindungsvorrichtung zur Herstellung einer lösbaren Verbindung verbunden. Dabei ist der eine Teil der Verbindungsvorrichtung mit dem Zulauf verbunden und der andere Teil der Verbindungsvorrichtung ist mit der Leitung verbunden. Die beiden Teile der Verbindungsvorrichtung sind vorzugsweise ohne Werkzeug trennbar. Die Oberflächen der mit den Flüssigkeiten in Berührung kommenden Teile der Verbindungsvorrichtung sind dabei im Wesentlichen glatt ausgestaltet, wobei glatt in dem Sinne zu verstehen ist, dass die Oberflächen frei von Ecken und Kanten sind, so dass keine Rückstände von Flüssigkeiten in der Verbindungsvorrichtung verbleiben. Der zulaufseitige Teil der Verbindungsvorrichtung ist durch einen Stopfen verschließbar, wobei der Stopfen wie der leitungsseitige Teil der Verbindungsvorrichtung ausgebildet ist, aber keine Möglichkeit für die Durchleitung der Flüssigkeiten bietet, so dass der Stopfen den Zulauf hermetisch verschließt. In einer Weiterbildung der Erfindung erfolgt das Verschließen des Zulaufs bei Trennung der lösbaren Verbindung automatisch durch einen im zulaufseitigen Teil der Verbindungsvorrichtung enthaltenen Mechanismus mit einer Klappe.

In einer weiteren ganz besonders bevorzugten Ausgestaltung der Erfindung ist der Fußbodenablauf derart in dem Boden eines Operationsraums versenkt, dass eine ebene Seite des Fußbodenablaufs bündig mit dem Bodenbelag des Operationsraums abschließt. Die ebene Seite umfasst sowohl den zulaufseitigen Teil der Verbindungsvorrichtung als auch den Deckel. Auch bei in den zulaufseitigen Teil der Verbindungsvorrichtung eingesetztem Stopfen schließt der Fußbodenabfluss bündig mit dem Boden ab. Die Stabilität und Robustheit des Fußbodenablaufs ist so ausgelegt, dass der Fußbodenablauf den gleichen mechanischen Belastungen standhalten kann, die auch bei Benutzung eines Operationsraums auf den Boden des Operationsraums wirken. Zur hermetischen Abdichtung des sich zwangsläufig zwischen dem Fußbodenablauf und dem Bodenbelag ergebenden Spalts ist am Fußbodenablauf ein Dichtmittel installiert. Der Fußbodenablauf stellt weder ein Hindernis für Personen noch für auf Rollen gelagerte Gegenstände dar. Auch wird der Aufwand für das Reinigen und Desinfizieren eines Operationsraums durch den Fußbodenablauf nicht erhöht und die Hygiene nicht beeinträchtigt. Die Positionierung des Deckels auf der mit dem Bodenbelag bündig abschließenden Seite des Fußbodenabflusses gewährt einen einfachen Zugang zum Innenraum des Fußbodenabflusses, wodurch es beispielsweise möglich ist, die elektrische Ultraviolettlichtquelle bei nicht mehr ausreichender Intensität des emittierten Ultraviolettlichtes auszutauschen.

Bei einer anderen bevorzugten Ausgestaltung der Erfindung ist im Behälter eine einen bei entferntem Deckel entnehmbaren mechanischen Filter umfassende Auffangvorrichtung zum Auffangen der in den Flüssigkeiten mitgeführten Feststoffe installiert. Die Auffangvorrichtung verhindert die Anlagerung von Feststoffen auf dem Boden des Behälters und erleichtert auf diese Weise die Entsorgung der Feststoffe.

Im Einzelnen gibt es nun verschiedene Möglichkeiten, den erfindungsgemäßen Fußbodenabfluss auszugestalten und weiterzubilden. Dazu wird verwiesen auf die dem Patentanspruch 1 nachgeordneten Patentansprüche und auf die Beschreibung bevorzugter Ausführungsbeispiele in Verbindung mit der Zeichnung. In der Zeichnung zeigen in geschnittener Ansicht
- Fig. 1: ein Ausführungsbeispiel des erfindungsgemäßen Fußbodenabflusses in perspektivischer Darstellung,
- Fig. 2: den Fußbodenabfluss aus Fig. 1 in Seitenansicht und
- Fig. 3: den Fußbodenabfluss aus Fig. 1 in Aufsicht.

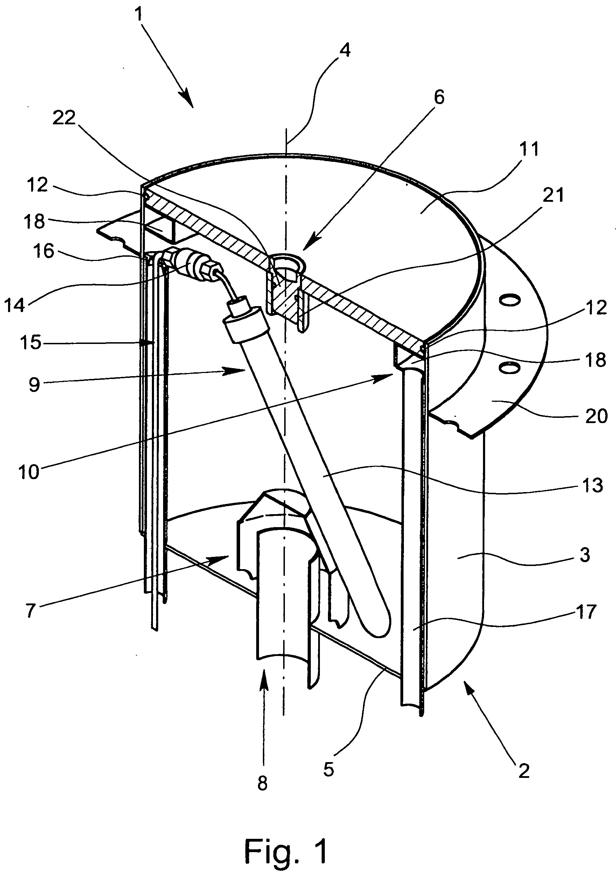

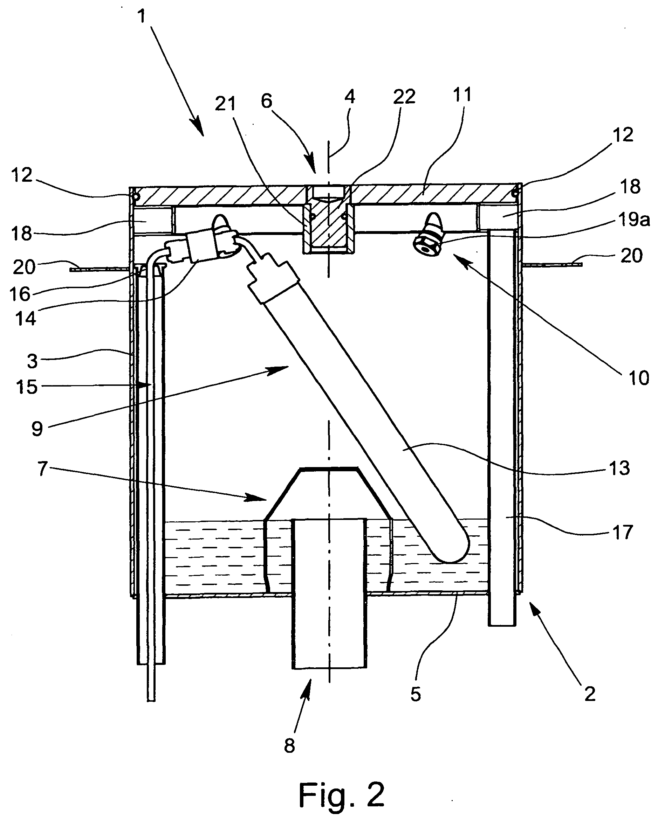

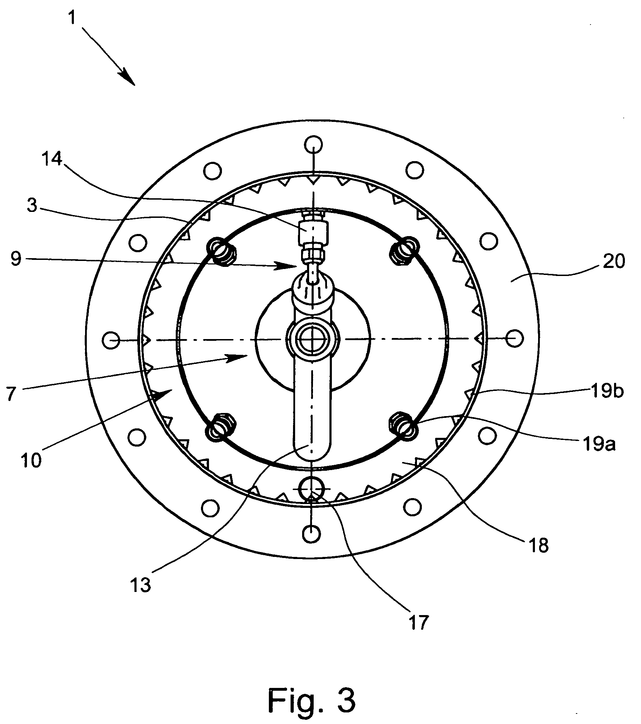

In den Figuren 1 bis 3 ist ein im Wesentlichen aus rostfreien Edelstählen hergestellter erfindungsgemäßer Fußbodenabfluss 1 mit einem topfförmigen Behälter 2 dargestellt, wobei der rohrförmige Mantel 3 des Behälters 2 rotationssymmetrisch zu einer Achse 4 ist und der kreisrunde scheibenförmige Boden 5 des Behälters 2 konzentrisch zu der Achse 4 angeordnet ist und mit dem Mantel 3 ringsum verschweißt ist.

Die in einem Operationsraum anfallenden Flüssigkeiten, insbesondere mit Krankheitserregern kontaminierte Flüssigkeiten, werden dem Fußbodenabfluss 1 über einen Zulauf 6 zugeführt und über einen in dem Boden 5 angeordneten und mit einem Gasverschluss 7 ausgestatteten Ablauf 8 abgeführt. Der Zulauf 6 und der Ablauf 8 liegen auf der Achse 4 und bei bestimmungsgemäßer Installation des Fußbodenabflusses 1 verläuft die Achse 4 im Wesentlichen derart parallel zur Wirkungsrichtung des Erdschwerefeldes, dass der Zulauf 6 oberhalb des Ablaufs 8 liegt.

Der Gasverschluss 7 ist als ein Geruchsverschluss ausgebildet, der zwei wesentliche Komponenten umfasst. Die eine Komponente ist ein zur Achse 4 rotationssymmetrisches Rohr, das durch den Boden 5 geführt in den Innenraum des Behälters 2 ragt und mit dem Boden 5 ringsum verschweißt ist. Die Höhe des oberen Randes des Rohres im Innenraum des Behälters 2 über dem Boden 5 bestimmt die Höhe des Standes der im Behälter 2 verbleibenden Flüssigkeiten. Die andere Komponente ist eine zur Achse 4 rotationssymmetrische Kappe mit Öffnungen unterhalb des Standes der in dem Behälter 2 verbleibenden Flüssigkeiten. Die hermetische Isolation des Innenraums des Behälters 2 gegenüber dem Ablauf 8 erfolgt durch die im Behälter 2 verbleibenden Flüssigkeiten. Der Gasverschluss 7 verhindert eine Geruchsbelastung aus den an den Ablauf 8 angeschlossenen Leitungen.

Im Innenraum des Behälters 2 befinden sich eine Bestrahlungsvorrichtung 9 zur Desinfektion der im Innenraum verbleibenden Flüssigkeiten durch Ultraviolettstrahlung und eine Spülvorrichtung 10 zur Reinigung des Innenraums des Behälters 2, wobei die Bestrahlungsvorrichtung 9 und die Spülvorrichtung 10 über die offene Seite des topfförmigen Behälters 2 zugänglich sind. Ein kreisrunder und ebener scheibenförmiger Deckel 11 verschließt die offene Seite des topfförmigen Behälters 2, wobei der Durchmesser des Deckels 11 geringfügig kleiner ist als der Innendurchmesser des Mantels 3 und die äußere ebene Seite des eingesetzten Deckels 11 mit dem oberen Rand des Mantels 3 in einer Ebene liegt.

Ein elastisches ringförmiges Dichtmittel 12 ist in eine über den Umfang des Deckels 11 verlaufende Nut eingebracht, deren Tiefe geringer als der Durchmesser des Querschnitts des Dichtmittels 12 ist, so dass der Außendurchmesser des Deckels 11 mit eingebrachtem Dichtmittel 12 größer ist als der Innendurchmesser des Mantels 3. Daher übt das Dichtmittel 12 bei eingesetztem Deckel 11 eine Kraft auf die Innenseite des Mantels 3 und auf die Nut des Deckels 11 aus, welche den Deckel 11 zum einen fixiert und zum anderen die hermetische Abdichtung zwischen Deckel 11 und Mantel 3 gewährleistet. Die Nut über den Umfang des Deckels 11 verläuft unmittelbar unterhalb der außen liegenden Seite des Deckels 11, so dass der von dem Deckel 11, dem Mantel 3 und dem Dichtmittel 12 begrenzte ringförmige Spalt ein kleinstmögliches Volumen besitzt, in dem sich die Flüssigkeiten und andere Rückstände ansammeln können.

Die Bestrahlungsvorrichtung 9 umfasst eine hermetisch abgedichtete Niederdruck-Quecksilberdampflampe mit einer Wellenlänge von 254 nm als elektrische Ultraviolettlichtquelle 13, eine lösbare Verbindung 14 und eine elektrische Energiezuführung 15, wobei das Kabel der Energiezuführung 15 durch ein Leerrohr geführt ist. Das Leerrohr gelangt dabei durch den Boden 5 in den Innenraum des Behälters 2, verläuft parallel zur Achse 4 an dem Mantel 3 entlang und endet oberhalb des Flüssigkeitsstands im Behälter 2, wobei es mit dem Boden 5 zur Abdichtung und dem Mantel für mechanische Stabilität verschweißt ist. Die Öffnung des Leerrohres, durch die das Kabel in den Innenraum des Behälters 2 tritt, ist durch ein Dichtmittel 16 hermetisch abgeschlossen. Die Verbindung 14 verbindet die Leitung mit der Ultraviolettlichtquelle 13 elektrisch und mechanisch und ist gegen äußere Einwirkungen entsprechend der Norm DIN EN 60529 IP68 geschützt. Da die Verbindung 14 ohne Werkzeug lösbar ist, kann die Ultraviolettlichtquelle 13 auf einfache Weise nach Entfernung des Deckels 11 ausgetauscht werden.

Die Spülvorrichtung 10 umfasst eine Spülmittelzuführung 17, eine Spülmittelverteilung 18 und zwei unterschiedliche Arten von Spülmittelaustritten 19a und 19b, wobei die Spülmittelaustritte 19 in der Spülmittelverteilung 18 angeordnet sind und von dieser mit einem Spülmittel versorgt werden. Die Spülmittelverteilung 18 ist ein konzentrisch um die Achse 4 im Innenraum des Behälters 2 angeordneter hohler Ring mit rechteckigem Querschnitt, der mit dem Mantel 3 verschweißt ist. Der Deckel 11 liegt auf der Spülmittelverteilung 18 auf, so dass auf den Deckel 11 parallel zur Achse 4 in Richtung der Erdschwerebeschleunigung wirkende Kräfte von der Spülmittelverteilung 18 aufgenommen und in den Mantel 3 abgeleitet werden. Die Spülmittelverteilung 18 versorgt demnach nicht nur die Spülmittelaustritte 19 mit dem Spülmittel, sondern trägt auch den Deckel 11, wodurch die mechanische Komplexität und der Herstellungsaufwand des Fußbodenablaufs 1 reduziert werden.

Darüber hinaus geht mit der Platzierung der Spülmittelverteilung 18 unmittelbar unterhalb des Deckels 11 auch eine Platzierung der Spülmittelaustritte 19 nahe des Deckels 11 einher, wodurch das Abspülen von Rückständen unterstützt wird, da das Spülmittel durch die Erdschwerebeschleunigung in Richtung auf den Boden 5 läuft und dabei Rückstände abtransportiert. Daher ist kein hoher Spülmitteldruck für die Reinigung erforderlich. Die vier Spülmittelaustritte 19a dienen im Wesentlichen der Reinigung der Ultraviolettlichtquelle 13 und des aus den Flüssigkeiten herausragenden Teils des Gasverschlusses 7. Dagegen dienen die Spülmittelaustritte 19b im Wesentlichen der Reinigung der Innenseite des Mantels 3.

Die Spülmittelzuführung 17 ist ein rundes Rohr, dass durch den Boden 5 in den Innenraum des Behälters 2 gelangt, parallel zur Achse 4 an dem Mantel 3 entlang verläuft und in der Spülmittelverteilung 18 endet, wobei das Rohr mit dem Boden 5 zur Abdichtung des Innenraums des Behälters 2 und mit der Spülmittelverteilung 18 verschweißt ist. Nicht eingezeichnet ist hier die außerhalb des Behälters 2 liegende Anschlussvorrichtung der Spülmittelzufiihrung 17 an eine Spülmittelquelle.

Durch die Anordnung sowohl der Bestrahlungsvorrichtung 9 als auch der Spülvorrichtung 10 im Innenraum des Behälters 2 und die Durchführung des Rohres des Ablaufs 8, des Leerrohres der Energiezuführung 15 und der Spülmittelzuführung 17 durch den Boden 5 bleibt die äußere rohrförmige Form des Mantels 3 erhalten und ununterbrochen. Daher kann die Installation in einem auf einfache Weise anzubringenden kreisrunden Loch in der Decke des Bodens des Operationsraums erfolgen, wobei der Durchmesser des Lochs geringfügig größer ist als der Außendurchmesser des Mantels 3, so dass der Behälter 2 in das Loch eingebracht werden kann. Der Fußbodenabfluss 1 ruht dabei auf einem rings um die Achse 4 angeordneten und senkrecht zur Achse 4 ausgerichteten Mauerkragen 20, der mit der äußeren Wand des Mantels 3 verschweißt ist, auf der Oberseite der Decke des Bodens des Operationsraums. Der Mauerkragen 20 wird mittels Schraubverbindungen mechanisch fest mit der Decke verbunden, wobei die Löcher für die Schrauben im Mauerkragen 20 vorgesehen sind. Ein auf die Decke aufgebrachter Belag - z.B. Estrich - umschließt den Mauerkragen 20 und ein auf den Belag aufgebrachter und den hygienischen Anforderungen eines Operationsraums genügende Bodenbelag liegt in einer Ebene mit dem oberen Rand des Mantels. Der seitlich zwischen Mantel und Bodenbelag verbleibende Spalt ist durch ein hier nicht dargestelltes Dichtungsmittel hermetisch abgedichtet.

Der Zulauf 6 und eine Leitung für die Zuführung der Flüssigkeiten in den Fußbodenablauf 1 sind zur Herstellung einer lösbaren Verbindung mit einer Verbindungsvorrichtung 21 ausgestattet, die einen O-Ringverschluss aufweist. Im getrennten Zustand verbleibt ein Teil der Verbindungsvorrichtung 21 am Zulauf 6 und der andere Teil der Verbindungsvorrichtung 21, der auch den O-Ringverschluss umfasst, verbleibt an der Leitung. Im verbundenen Zustand sind die beiden Teile der Verbindungsvorrichtung 21 hermetisch dicht gegenüber der Umgebung miteinander verbunden. Die Oberflächen der mit den Flüssigkeiten in Verbindung kommenden Teile der Verbindungsvorrichtung 21 sind im Wesentlichen glatt und derart im Bezug auf die Erdschwerebeschleunigung ausgerichtet, dass Flüssigkeitsrückstände in der Verbindungsvorrichtung 21 in den Behälter 2 abfließen.

Werden keine Flüssigkeiten in den Fußbodenabfluss 1 eingeleitet, so wird der Zulauf 6 mit einem Stopfen 22 hermetisch verschlossen. Der Stopfen 22 gleicht dem im getrennten Zustand an der Leitung verbleibenden Teil der Verbindungsvorrichtung 21 mit der Ausnahme, dass der Stopfen 22 verschlossen ist, so dass bei eingesetztem Stopfen 22, eingesetztem Deckel 11 und einem Stand der im Behälter 2 verbleibenden Flüssigkeiten oberhalb der Öffnungen in der Kappe des als Geruchsverschluss ausgebildeten Gasverschlusses der Innenraum des Behälters 2 hermetisch abgeschlossen ist.

Der Bodenbelag des Operationsraums bildet mit dem oberen Rand des Mantels 3, der Oberseite des Deckels 11 und dem Stopfen 22 eine ebene Fläche, die, insbesondere für auf Rollen gelagerte Gegenstände, kein Hindernis darstellt. Der Deckel 11 hält den gleichen mechanischen Belastungen wie der Boden des Operationsraums stand. Durch den erfindungsgemäßen Fußbodenabfluss 1 werden weder Reinigungs- noch Desinfektionsaufwand für den Boden des Operationsraums erhöht.

## Patentansprüche

1. Fußbodenabfluss (1) zur Installation in einem Operationsraum und zur Abführung von in dem Operationsraum anfallenden Flüssigkeiten, insbesondere von den mit Krankheitserregern kontaminierten Flüssigkeiten, mit einem geschlossenen Behälter (2), mindestens einem Zulauf (6) für die Zuführung der Flüssigkeiten in den Behälter (2) hinein, einem mit einem Gasverschluss (7) ausgestatteten Ablauf (8) für die Abführung der Flüssigkeiten aus dem Behälter (2) heraus, einer Bestrahlungsvorrichtung (9) für die Bestrahlung des Innenraums des Behälters (2) mit Ultraviolettlicht zur Desinfektion,
**dadurch gekennzeichnet,**
**dass** eine mit einer Spülflüssigkeit arbeitende Spülvorrichtung (10) für die Spülung des Innenraums des Behälters (2) zur Reinigung umfasst ist, und dass der Zulauf (6) und eine Leitung für die Zuführung der Flüssigkeiten in den Fußbodenabfluss (1) mittels einer Verbindungsvorrichtung (21) zur Herstellung einer lösbaren Verbindung miteinander verbindbar sind, wobei die Oberflächen der mit den Flüssigkeiten in Berührung kommenden Teile der Verbindungsvorrichtung (21) glatt sind.

2. Fußbodenabfluss (1) nach Anspruch 1, **dadurch gekennzeichnet, dass** der Gasverschluss (7) als ein Geruchsverschluss ausgebildet ist.

3. Fußbodenabfluss (1) nach Anspruch 1 oder 2, **dadurch gekennzeichnet,**
**dass** der Behälter (2) einen lösbar verbindbaren Deckel (11) aufweist, wobei die Verbindung zwischen Behälter (2) und Deckel (11) durch ein am Deckel angeordnetes Dichtmittel (12) hermetisch dicht ist.

4. Fußbodenabfluss (1) nach einem der Ansprüche 1 bis 3, **dadurch gekennzeichnet, dass** ein Sensor zur Messung der Intensität des von der Bestrahlungsvorrichtung (9) emittierten Ultraviolettlichts in dem Behälter (2) angeordnet ist.

5. Fußbodenabfluss (1) nach einem der Ansprüche 1 bis 4, **dadurch gekennzeichnet, dass** die Bestrahlungsvorrichtung (9) eine elektrische Ultraviolettlichtquelle (13) und eine Energiezuführung (15) umfasst, wobei die Energiezuführung (15) außerhalb oder innerhalb des Behälters (2) angeordnet ist.

6. Fußbodenabfluss (1) nach einem der Ansprüche 1 bis 5, **dadurch gekennzeichnet, dass** die Spülvorrichtung (10) eine Spülmittelzuführung (17), eine Spülmittelverteilung (18) und zumindest einen Spülmittelaustritt (19) umfasst, wobei die Spülmittelzuführung (17) und / oder die Spülmittelverteilung (18) außerhalb oder innerhalb des Behälters (2) angeordnet sind.

7. Fußbodenabfluss (1) nach einem der Ansprüche 1 bis 6, **dadurch gekennzeichnet, dass** der Zulauf (6) hermetisch verschließbar durch einen Stopfen (22) oder automatisch verschließbar bei Trennung der Verbindungsvorrichtung (21) durch eine Vorrichtung im Fußbodenabfluss (1) ist.

8. Fußbodenabfluss (1) nach einem der Ansprüche 1 bis 7, **dadurch gekennzeichnet, dass** der Innenraum des Behälters (2) ohne Berücksichtigung des Zulaufs (6) hermetisch abgeschlossen ist.

9. Fußbodenabfluss (1) nach einem der Ansprüche 1 bis 8, **dadurch gekennzeichnet, dass** ein Dichtmittel den zwangsläufig beim Einlassen des Fußbodenabflusses (1) in den Boden des Operationsraums verbleibenden Spalt zwischen dem Fußbodenabfluss (1) und dem Boden hermetisch abdichtet.

10. Fußbodenabfluss (1) nach einem der Ansprüche 1 bis 9, **dadurch gekennzeichnet, dass** beim vollständigen Einlassen des Fußbodenablaufs (1) in den Boden des Operationsraums eine ebene Seite des Fußbodenablaufs (1) bündig mit dem Boden des Operationsraums abschließt und diese Seite des Fußbodenablaufs (1) den Zulauf (6) und den Deckel (11) umfasst.

11. Fußbodenabfluss (1) nach einem der Ansprüche 1 bis 10, **dadurch gekennzeichnet, dass** im Innenraum des Behälters (2) eine Auffangvorrichtung zum Auffangen von in den Flüssigkeiten enthaltenen Festkörpern angeordnet ist.

## Claims

1. Floor drain (1) for installation in an operating room and for discharge of fluids accumulating in the operating room, in particular of fluids contaminated with pathogens, having a closed container (2), at least one inlet (6) for leading the fluid into the container (2), an outlet (8) equipped with a gas trap (7) for discharge of fluids from the container (2), an irradiation device (9) for irradiating the interior of the container (2) with ultraviolet light for disinfection
**characterized in**
**that** a rinsing device (10) is incorporated to rinse the interior of the container (2) for cleaning, and that the inlet (6) and a pipe for leading the fluid into the floor drain (1) can be connected to one another by means of a connecting device (21) for producing a detachable connection, wherein the surfaces of the parts of the connecting device (21) coming into contact with the fluids are smooth.

2. Floor drain (1) according to claim 1, **characterized in that** the gas trap (7) is designed as an odor trap.

3. Floor drain (1) according to claim 1 or 2, **characterized in that** the container (2) has a removably connectable cover (11), wherein the connection between container (2) and cover (11) is hermetically sealed by means of a sealant (12) arranged on the cover.

4. Floor drain (1) according to any one of claims 1 to 3, **characterized in**
**that** a sensor for measuring the intensity of the ultraviolet light emitted by the irradiation device (9) is arranged in the container (2).

5. Floor drain (1) according to any one of claims 1 to 4, **characterized in**
**that** the irradiation device (9) comprises an electric ultraviolet light source (13) and a power supply (15), wherein the power supply (15) is arranged inside or outside of the container (2).

6. Floor drain (1) according to any one of claims 1 to 5, **characterized in**
**that** the rinsing device (10) comprises a rinsing agent feed (17), a rinsing agent distribution (18), and at least one rinsing agent discharge (19), wherein the rinsing agent feed (17) and/or the rinsing agent distribution (18) are arranged outside or inside the container (2).

7. Floor drain (1) according to any one of claims 1 to 6, **characterized in**
**that** the inlet (6) can be hermetically closed by a plug (22) or can be automatically closed in that the connection device (21) is separated by means of a device in the floor drain (1).

8. Floor drain (1) according to any one of claims 1 to 7, **characterized in**
**that** the interior of the container (2) is hermetically closed without taking the inlet (6) into consideration.

9. Floor drain (1) according to any one of claims 1 to 8, **characterized in**
**that** a sealant hermetically seals the gap inevitably remaining between the floor drain (1) and the floor when inserting the floor drain (1) in the floor of the operating room.

10. Floor drain (1) according to any one of claims 1 to 9, **characterized in**
**that** when completely inserting the floor drain (1) in the floor of the operating room, a flat side of the floor drain (1) ends flush with the floor of the operating room and this side of the floor drain (1) comprises the inlet (6) and the cover (11).

11. Floor drain (1) according to any one of claims 1 to 10, **characterized in that** a retaining device for retaining solids contained in the fluids is arranged in the interior of the container (2).

## Revendications

1. Evacuation de plancher (1) destinée à être installée dans une salle d'opération pour évacuer des liquides produits dans la salle d'opération, en particulier des liquides contaminés par des vecteurs de maladies, et comprenant un récipient fermé (2), au moins une amenée (6) qui permet l'amenée des liquides dans le récipient (2), une évacuation (8) dotée d'une fermeture à gaz (7) permet d'évacuer les liquides hors du récipient (2), un ensemble d'irradiation (9) qui irradie l'espace intérieur du récipient (2) par de la lumière ultraviolette en vue de sa désinfection, **caractérisée en ce que**
un ensemble de rinçage (10) travaillant avec un liquide de rinçage pour le rinçage de l'espace intérieur du récipient (2) est inclus pour le nettoyage,
**en ce que** l'amenée (6) et un conduit d'amenée des liquides dans l'évacuation de plancher (1) peuvent être reliés mutuellement au moyen d'un ensemble de liaison (21) qui établit une liaison libérable et
**en ce que** les surfaces de l'ensemble de liaison (21) entrant en contact avec les liquides sont lisses.

2. Evacuation de plancher (1) selon la revendication 1, **caractérisée en ce que** la fermeture à gaz (7) est configurée comme siphon anti-odeurs.

3. Evacuation de plancher (1) selon les revendications 1 ou 2, **caractérisée en ce que** le récipient (2) présente un couvercle (11) apte à être raccordé de manière libérable, la liaison entre le récipient (2) et le couvercle (11) étant fermée hermétiquement par un moyen d'étanchéité (12) disposé sur le couvercle.

4. Evacuation de plancher (1) selon l'une des revendications 1 à 3, **caractérisée en ce qu'**un capteur de mesure de l'intensité de la lumière ultraviolette émise par l'ensemble d'irradiation (9) est disposé dans le récipient (2).

5. Evacuation de plancher (1) selon l'une des revendications 1 à 4, **caractérisée en ce que** l'ensemble d'irradiation (9) comporte une source (13) de lumière ultraviolette et une amenée d'énergie (15), l'amenée d'énergie (15) étant disposée à l'extérieur ou à l'intérieur du récipient (2).

6. Evacuation de plancher (1) selon l'une des revendications 1 à 5, **caractérisée en ce que** le dispositif de rinçage (10) comporte une amenée (17) d'agent de rinçage, une répartition (18) d'agent de rinçage et au moins une sortie (19) d'agent de rinçage, l'amenée (17) d'agent de rinçage et/ou la répartition (18) d'agent de rinçage étant disposées à l'extérieur ou à l'intérieur du récipient (2).

7. Evacuation de plancher (1) selon l'une des revendications 1 à 6, **caractérisée en ce que** l'amenée (6) peut être fermée hermétiquement par un bouchon (22) ou peut être fermée automatiquement par séparation de l'ensemble de liaison (21) par un ensemble prévu dans l'évacuation de plancher (1).

8. Evacuation de plancher (1) selon l'une des revendications 1 à 7, **caractérisée en ce qu'**à l'exception de l'amenée (6), l'espace intérieur du récipient (2) peut être fermé hermétiquement.

9. Evacuation de plancher (1) selon l'une des revendications 1 à 8, **caractérisée en ce qu'**un moyen d'étanchéité ferme hermétiquement l'interstice qui reste obligatoirement entre l'évacuation de plancher (1) et le sol lorsque l'évacuation de plancher (1) est placée dans le sol de la salle d'opération.

10. Evacuation de plancher (1) selon l'une des revendications 1 à 9, **caractérisée en ce qu'**un côté plan de l'évacuation de plancher (1) se raccorde à chant au sol de la salle d'opération lorsque l'évacuation de plancher (1) est entièrement placée dans le sol de la salle d'opération et **en ce que** ce côté de l'évacuation de plancher (1) comporte l'amenée (6) et le couvercle (11).

11. Evacuation de plancher (1) selon l'une des revendications 1 à 10, **caractérisée en ce qu'**un ensemble de piégeage qui piège des corps solides contenus dans les liquides est disposé dans l'espace intérieur du récipient (2).
